# EUROPEAN PATENT APPLICATION

(11) **EP 4 510 013 A1**
(43) Date of publication of application: **19.02.2025**
(21) Application number: 22937465.7
(22) Date of filing: 14.04.2022
(51) Int. Cl.: G06F 17/13, G06F 17/17

(54) **INFORMATION PROCESSING PROGRAM AND INFORMATION PROCESSING METHOD**

(71) Applicant: FUJITSU LIMITED, Kawasaki-shi, Kanagawa 211-8588 (JP)
(72) Inventor: OHTA, Eiji, Kawasaki-shi, Kanagawa 211-8588 (JP)
(74) Representative: Hoffmann Eitle
(86) International application number: PCT/JP2022/017849
(87) International publication number: WO 2023/199486

(57) **Abstract**

An information processing device (100) stores a function (101) that enables calculation of a parameter. The information processing device (100) sets at least any one of a condition A or a condition B as an end condition. The condition A indicates that the number of upper digits whose values match between a characteristic value corresponding to a solution of the parameter calculated last time and a characteristic value corresponding to a solution of the parameter calculated this time is equal to or greater than a threshold a. The condition B indicates that a degree of variation between a difference between the solution of the parameter calculated last time and a solution of the parameter calculated a time before last, and a difference between the solution of the parameter calculated this time and the solution of the parameter calculated last time is equal to or smaller than a threshold b. The information processing device (100) repeatedly performs a specific operation of calculating a solution of the parameter using the function (101) until the end condition is satisfied.

## Description

### TECHNICAL FIELD

The present invention relates to an information processing program and an information processing method.

### BACKGROUND ART

Conventionally, in a field of material development, there is a technology of calculating an electron density by a numerical analysis method. The numerical analysis method is, for example, a self consistent field method. For example, in a case where the electron density is calculated by the self consistent field method, an operation of calculating the electron density using a wave function is repeated until it is determined that the electron density has converged. For example, in a case where the electron density is calculated by the self consistent field method, when the number of atoms is N, a calculation amount is O(N^3).

As prior art, for example, there is a technology of repeatedly calculating a value indicating a spin state of an electron included in a substance by using a value indicating a virtual magnetic field. Furthermore, for example, there is a technology of determining convergence by comparing a result obtained by previous calculation with a result calculated this time for energy and a one-electron reduced density matrix. Furthermore, for example, there is a technology of performing end determination based on comparison between a total energy value and a convergence determination value in the self consistent field method.

### CITATION LIST

### PATENT DOCUMENT

Patent Document 1: Japanese Laid-open Patent Publication No. 2015-141533
Patent Document 2: Japanese Laid-open Patent Publication No. 2006-277582
Patent Document 3: U.S. Patent No. 6106562

### SUMMARY OF INVENTION

### TECHNICAL PROBLEM

However, the prior art has a problem of causing an increase in a calculation amount when the electron density is calculated by the numerical analysis method. For example, in a case where the electron density is calculated by the self consistent field method, it may be difficult to determine that the electron density has converged and the number of times of repeating the operation may increase, and the calculation amount increases.

In one aspect, an object of the present invention is to suppress an increase in a calculation amount.

### SOLUTION TO PROBLEM

According to one embodiment, there are proposed an information processing program and an information processing method for repeatedly performing a specific operation of calculating a solution of a parameter by using a function until the number of upper digits whose values match between a characteristic value that corresponds to a solution of the parameter calculated last time and a characteristic value that corresponds to a solution of the parameter calculated this time becomes equal to or greater than a threshold.

Furthermore, according to one embodiment, there are proposed an information processing program and an information processing method for repeatedly performing a specific operation of calculating a solution of a parameter by using a function until a degree of variation between a difference between a solution of the parameter calculated last time and a solution of the parameter calculated a time before last, and a difference between a solution of the parameter calculated this time and the solution of the parameter calculated last time becomes equal to or smaller than a threshold.

### ADVANTAGEOUS EFFECTS OF INVENTION

In one aspect, it becomes possible to suppress an increase in a calculation amount.

### BRIEF DESCRIPTION OF DRAWINGS

FIG. 1 is an explanatory diagram illustrating one example of an information processing method according to an embodiment.
FIG. 2 is an explanatory diagram illustrating an example of an information processing system 200.
FIG. 3 is a block diagram illustrating a hardware configuration example of an information processing device 100.
FIG. 4 is a block diagram illustrating a functional configuration example of the information processing device 100.
FIG. 5 is an explanatory diagram illustrating a first operation example of the information processing device 100.
FIG. 6 is a flowchart illustrating an example of an overall processing procedure in the first operation example.
FIG. 7 is a flowchart illustrating an example of an operation processing procedure in the first operation example.
FIG. 8 is an explanatory diagram illustrating a second operation example of the information processing device 100.
FIG. 9 is a flowchart illustrating an example of an overall processing procedure in the second operation example.
FIG. 10 is a flowchart illustrating an example of an operation processing procedure in the second operation example.
FIG. 11 is an explanatory diagram illustrating a comparative example of a calculation result with a prior method.
FIG. 12 is an explanatory diagram illustrating a comparative example of a speed-up rate with the prior method.
FIG. 13 is an explanatory diagram illustrating a display example.

### DESCRIPTION OF EMBODIMENTS

Hereinafter, an embodiment of an information processing program and an information processing method according to the present invention will be described in detail with reference to the drawings.

### (One Example of Information Processing Method According to Embodiment)

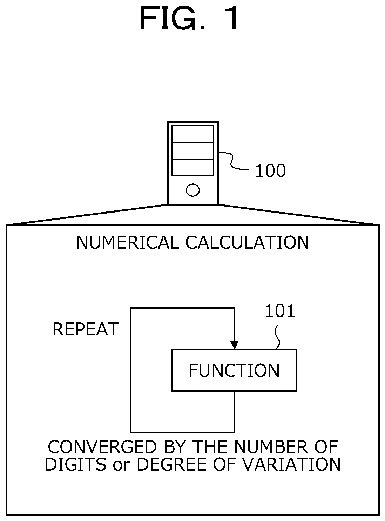

FIG. 1 is an explanatory diagram illustrating one example of the information processing method according to the embodiment. An information processing device 100 is a computer for suppressing an increase in a calculation amount for numerical calculation. The information processing device 100 is, for example, a server, a personal computer (PC), or the like.

The numerical calculation corresponds to, for example, an iterative solution. The iterative solution is, for example, a method of repeatedly performing a specific operation until it is determined that a solution has converged. The convergence is, for example, a state where a difference between a solution calculated last time and a solution calculated this time is equal to or smaller than a threshold. Specifically, the numerical calculation corresponds to a self consistent field method. In the self consistent field method, a specific operation of calculating an electron density at each point in a space using a wave function is repeatedly performed until it is determined that the electron density has converged. The convergence is, for example, a state where a difference between an electron density calculated last time and an electron density calculated this time is equal to or smaller than a threshold.

It is conceivable that the self consistent field method is used when, for example, it is attempted to calculate an electron density in a field of material development. Specifically, in the field of material development, the self consistent field method is used when it is attempted to accumulate material data by calculating an electron density according to a density functional theory for materials informatics for shortening a research period, and the like. For the density functional theory, for example, Reference Document 1 described below may be referred to.

Reference Document 1: Takao Tsuneda and Eiko Soejima, "Fundamentals of Density Functional Method", Book 11 (2012): 12

Here, the larger a scale of a problem to be calculated, the larger the calculation amount needed for the numerical calculation tends to be. For example, in the case of attempting to calculate the electron density, the larger the number of atoms, the larger the calculation amount needed for the self consistent field method tends to be. Moreover, there is a problem that a time needed until it is determined that a solution has converged becomes long depending on the problem to be calculated, which causes an increase in the number of times of performing the specific operation and an increase in the calculation amount.

Specifically, in the self consistent field method, in a case where a predetermined condition is satisfied, an increase in the number of times of repeating the specific operation of calculating the electron density at each point in the space is caused. The predetermined condition is that total electron energy transitions to a state of minute variation.

Specifically, the predetermined condition is that there is a point having a relatively small degree of influence on the total electron energy in the space, and the electron density at the point becomes difficult to converge. Specifically, the predetermined condition is that there is a point in the space where a variation rate of the difference between the electron density calculated last time and the electron density calculated this time is relatively small, and the electron density at the point becomes difficult to converge.

Thus, in the present embodiment, the information processing method capable of suppressing an increase in the calculation amount needed for the numerical calculation will be described. In the following description, the self consistent field method may be referred to as the "self consistent field (SCF) method".

In FIG. 1, the information processing device 100 stores a function 101 that enables calculation of a parameter. The parameter is, for example, an electron density. The function 101 is, for example, a Kohn-Sham equation that enables derivation of a one-electron wave function that enables calculation of the parameter.

(1-1) The information processing device 100 sets an end condition. For example, the information processing device 100 sets, as the end condition, a condition A indicating that the number of upper digits whose values match between a characteristic value corresponding to a solution of the parameter calculated last time and a characteristic value corresponding to a solution of the parameter calculated this time is equal to or greater than a threshold a. The characteristic value is, for example, total electron energy. The characteristic value may be, for example, the parameter itself. The characteristic value may be, for example, the electron density itself.

For example, the information processing device 100 sets, as the end condition, a condition B indicating that a degree of variation between a difference between the solution of the parameter calculated last time and a solution of the parameter calculated a time before last, and a difference between the solution of the parameter calculated this time and the solution of the parameter calculated last time is equal to or smaller than a threshold b. The information processing device 100 may set only one of the condition A and the condition B as the end condition. The information processing device 100 may set both the condition A and the condition B as the end conditions. The information processing device 100 may further set, as the end condition, a condition C indicating that the difference between the solution of the parameter calculated last time and the solution of the parameter calculated this time is equal to or smaller than a threshold c.

(1-2) The information processing device 100 repeatedly performs a specific operation of calculating a solution of the parameter using the function 101 until the end condition is satisfied. Specifically, the specific operation is a series of operations of calculating a solution of the parameter using the function 101 based on the solution of the parameter calculated last time. For example, the information processing device 100 sets an initial value of the parameter, and calculates a first solution of the parameter using the function 101. Thereafter, for example, the information processing device 100 repeatedly performs the series of operations of calculating a solution of the parameter using the function 101 based on the solution of the parameter calculated last time until the end condition is satisfied.

(1-3) In a case where the condition A is satisfied, the information processing device 100 outputs the solution of the parameter calculated this time without repeating the specific operation. An output format is, for example, display on a display, print output to a printer, transmission to an external device by a network I/F, storage to a storage area, or the like. For example, the information processing device 100 outputs the solution of the parameter calculated this time so that a user may refer to the solution. With this configuration, the information processing device 100 may suppress an increase in the calculation amount. For example, even when there is a point having a relatively small degree of influence on the total electron energy in a space, the information processing device 100 may suppress an increase in the number of times of repeating the specific operation.

In a case where the condition B is satisfied, the information processing device 100 outputs a notification indicating that the parameter is unconverged, without repeating the specific operation. For example, the information processing device 100 outputs the notification indicating that the parameter is unconverged so that a user may refer to the notification. With this configuration, the information processing device 100 may suppress an increase in the calculation amount. For example, the information processing device 100 may prevent the calculation amount from becoming enormous while there is a point in the space where a variation rate of a difference between an electron density calculated last time and an electron density calculated this time is relatively small and the parameter remains unconverged.

In a case where the condition C is satisfied, the information processing device 100 outputs the solution of the parameter calculated this time without repeating the specific operation. For example, the information processing device 100 outputs the solution of the parameter calculated this time so that a user may refer to the solution. With this configuration, the information processing device 100 may suppress an increase in the calculation amount. The information processing device 100 may make a relatively accurate solution of the parameter available. In this manner, the information processing device 100 may suppress an increase in the calculation amount, and may suppress an increase in a calculation time and a calculation load.

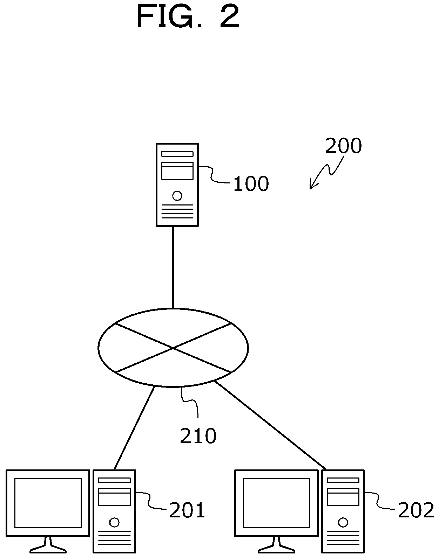

Here, a case has been described where the information processing device 100 operates independently. However, the embodiment is not limited to this. For example, there may be a case where a plurality of computers cooperates to implement a function as the information processing device 100. Specifically, there may be a case where the specific operation may be subjected to distributed processing by the plurality of computers. A specific example of the case where the plurality of computers cooperates will be described later with reference to FIG. 2, for example. For example, there may be a case where the function as the information processing device 100 is implemented in a cloud.

### (Example of Information Processing System 200)

Next, an example of an information processing system 200, to which the information processing device 100 illustrated in FIG. 1 is applied, will be described with reference to FIG. 2.

FIG. 2 is an explanatory diagram illustrating an example of the information processing system 200. In FIG. 2, the information processing system 200 includes the information processing device 100, a numerical calculation device 201, and a client device 202.

In the information processing system 200, the information processing device 100 and the client device 202 are coupled via a wired or wireless network 210. The network 210 is, for example, a local area network (LAN), a wide area network (WAN), the Internet, or the like. In the information processing system 200, the information processing device 100 and the numerical calculation device 201 are coupled via the wired or wireless network 210.

The information processing device 100 is a computer that manages numerical calculation. The information processing device 100 receives, for example, a calculation instruction from the client device 202. The calculation instruction specifies a problem to be calculated. The information processing device 100 sets an end condition. In response to the calculation instruction, the information processing device 100 repeatedly performs a specific operation until the end condition is satisfied.

For example, the information processing device 100 sets an initial value of an electron density, and calculates a first solution of the electron density using a one-electron wave function derived by solving a Kohn-Sham equation based on the initial value. Thereafter, for example, the information processing device 100 repeatedly performs a series of operations of calculating a solution of the electron density using the one-electron wave function derived by solving the Kohn-Sham equation based on a solution of the electron density calculated last time until the end condition is satisfied.

Here, for example, the information processing device 100 may perform distributed processing on the operations of calculating a solution of the electron density with the numerical calculation device 201. In a case where the end condition is satisfied, when the solution of the electron density has converged, the information processing device 100 transmits the solution of the electron density to the client device 202. In a case where the end condition is satisfied, when the solution of the electron density is unconverged, the information processing device 100 transmits a notification of unconvergence to the client device 202. The information processing device 100 is, for example, a server, a PC, or the like.

The numerical calculation device 201 is a computer that shares an operation of calculating a solution of an electron density. The numerical calculation device 201 is, for example, a server, a PC, or the like. The client device 202 is a computer that transmits a calculation instruction to the information processing device 100 based on operation input of a user. When receiving the solution of the electron density from the information processing device 100, the client device 202 outputs the solution so that the user may refer to the solution. When receiving a notification of unconvergence from the information processing device 100, the client device 202 outputs the notification so that the user may refer to the notification. The client device 202 is, for example, a PC, a tablet terminal, a smartphone, or the like.

In the following description, a case will be mainly described where the information processing device 100 operates independently.

### (Hardware Configuration Example of Information Processing Device 100)

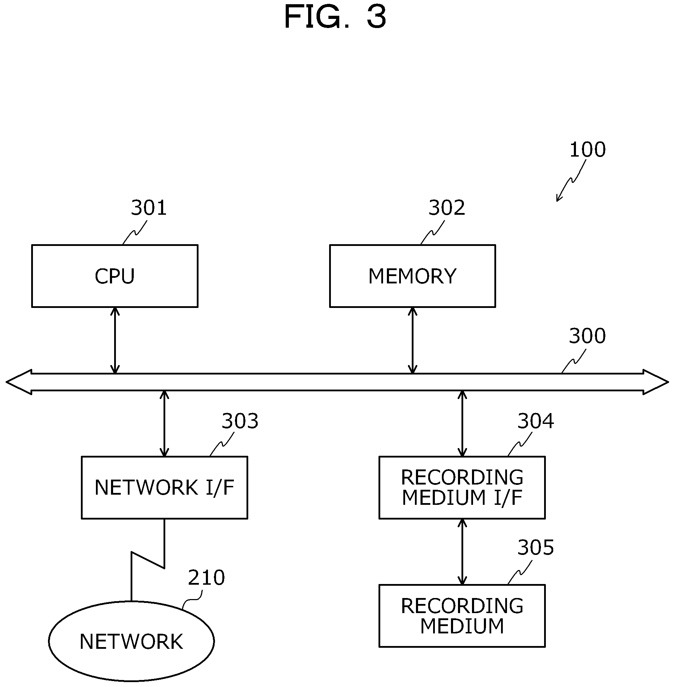

Next, a hardware configuration example of the information processing device 100 will be described with reference to FIG. 3.

FIG. 3 is a block diagram illustrating the hardware configuration example of the information processing device 100. In FIG. 3, the information processing device 100 includes a central processing unit (CPU) 301, a memory 302, a network interface (I/F) 303, a recording medium I/F 304, and a recording medium 305. Furthermore, the respective components are coupled to each other by a bus 300.

Here, the CPU 301 is in charge of overall control of the information processing device 100. The memory 302 includes, for example, a read only memory (ROM), a random access memory (RAM), a flash ROM, and the like. Specifically, for example, the flash ROM or the ROM stores various programs, and the RAM is used as a work area for the CPU 301. The programs stored in the memory 302 are loaded into the CPU 301 to cause the CPU 301 to execute coded processing.

The network I/F 303 is coupled to the network 210 through a communication line, and is coupled to another computer via the network 210. Then, the network I/F 303 takes control of an interface between the network 210 and the inside, and controls input and output of data to and from the another computer. The network I/F 303 is, for example, a modem, a LAN adapter, or the like.

The recording medium I/F 304 controls reading and writing of data from and to the recording medium 305 under the control of the CPU 301. Examples of the recording medium I/F 304 include a disk drive, a solid state drive (SSD), a universal serial bus (USB) port, or the like. The recording medium 305 is a nonvolatile memory that stores data written under the control of the recording medium I/F 304. Examples of the recording medium 305 include a disk, a semiconductor memory, a USB memory, or the like. The recording medium 305 may be attachable to and detachable from the information processing device 100.

The information processing device 100 may include, for example, a keyboard, a mouse, a display, a printer, a scanner, a microphone, a speaker, and the like in addition to the components described above. Furthermore, the information processing device 100 may include a plurality of the recording medium I/Fs 304 and a plurality of the recording media 305. Furthermore, the information processing device 100 does not have to include the recording medium I/F 304 or the recording medium 305.

### (Functional Configuration Example of Information Processing Device 100)

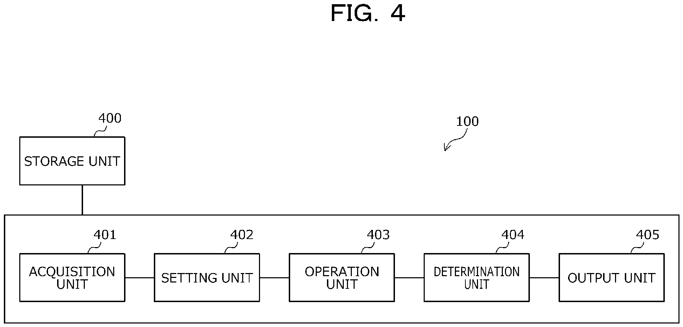

Next, a functional configuration example of the information processing device 100 will be described with reference to FIG. 4.

FIG. 4 is a block diagram illustrating the functional configuration example of the information processing device 100. The information processing device 100 includes a storage unit 400, an acquisition unit 401, a setting unit 402, an operation unit 403, a determination unit 404, and an output unit 405.

The storage unit 400 is implemented by, for example, a storage area such as the memory 302 or the recording medium 305 illustrated in FIG. 3. Hereinafter, a case where the storage unit 400 is included in the information processing device 100 will be described, but the embodiment is not limited to this. For example, there may be a case where the storage unit 400 is included in a device different from the information processing device 100, and the information processing device 100 may refer to content stored in the storage unit 400.

The acquisition unit 401 to the output unit 405 function as examples of a control unit. Specifically, the acquisition unit 401 to the output unit 405 implement functions thereof by, for example, causing the CPU 301 to execute a program stored in a storage area such as the memory 302 or the recording medium 305 illustrated in FIG. 3, or by the network I/F 303. A processing result of each functional unit is stored in, for example, a storage area such as the memory 302 or the recording medium 305 illustrated in FIG. 3.

The storage unit 400 stores various types of information to be referred to or updated in processing of each functional unit. The storage unit 400 stores a function for calculating a solution of a parameter. The parameter is, for example, an electron density. The function is, for example, a Kohn-Sham equation that enables derivation of a one-electron wave function that enables calculation of the parameter.

The acquisition unit 401 acquires various types of information to be used for processing of each functional unit. The acquisition unit 401 stores various types of the acquired information in the storage unit 400 or outputs various types of the acquired information to each functional unit. Furthermore, the acquisition unit 401 may output various types of information previously stored in the storage unit 400 to each functional unit. The acquisition unit 401 acquires various types of information based on, for example, operation input of a user. The acquisition unit 401 may receive various types of information from, for example, a device different from the information processing device 100.

The acquisition unit 401 acquires, for example, a calculation instruction. The calculation instruction includes, for example, information for specifying a problem to be calculated. Specifically, the acquisition unit 401 accepts input of the calculation instruction based on operation input from a user. Specifically, the acquisition unit 401 acquires the calculation instruction by receiving the calculation instruction from the another computer.

The acquisition unit 401 may accept a start trigger to start processing of any one of the functional units. The start trigger is, for example, predetermined operation input by a user. The start trigger may be, for example, reception of predetermined information from another computer. The start trigger may be, for example, output of predetermined information by any one of the functional units.

Specifically, the acquisition unit 401 accepts acquisition of the calculation instruction as a start trigger for starting processing of the setting unit 402, the operation unit 403, and the determination unit 404.

The setting unit 402 sets an end condition for repetition of a specific operation. The specific operation is, for example, a series of operations of calculating a solution of a parameter using a function. For example, the specific operation is a series of operations of calculating a solution of a parameter using a function based on a solution of the parameter calculated last time. Specifically, the specific operation corresponds to the SCF method.

For example, the setting unit 402 sets an end condition A indicating that the number of upper digits whose values match between a characteristic value corresponding to the solution of the parameter calculated last time and a characteristic value corresponding to a solution of the parameter calculated this time is equal to or greater than a first threshold. The first threshold is preset by a user, for example. The characteristic value is, for example, total electron energy. For example, in a case where the characteristic value corresponding to the solution of the parameter calculated last time is "5678" and the characteristic value corresponding to the solution of the parameter calculated this time is "5679", the number of digits is "3" since three values of upper digits "567" match.

Furthermore, for example, in a case where the characteristic value corresponding to the solution of the parameter calculated last time is "1234" and the characteristic value corresponding to the solution of the parameter calculated this time is "123.4", the number of digits is "0" since values existing in the same digits from upper digits do not match. With this configuration, the setting unit 402 may set a guideline for reducing the calculation amount. For example, the setting unit 402 may enable detection that a solution of the parameter has been accurately calculated.

For example, the setting unit 402 sets an end condition B indicating that a difference between the solution of the parameter calculated last time and the solution of the parameter calculated this time is equal to or smaller than a second threshold. The second threshold is, for example, a threshold different from the first threshold. The second threshold is preset by a user, for example. With this configuration, the setting unit 402 may set a guideline for reducing the calculation amount. For example, the setting unit 402 may enable detection that a solution of the parameter has been accurately calculated.

For example, the setting unit 402 sets an end condition C indicating that a degree of variation between a difference between the solution of the parameter calculated last time and a solution of the parameter calculated a time before last, and the difference between the solution of the parameter calculated this time and the solution of the parameter calculated last time is equal to or smaller than a third threshold. The third threshold is, for example, a threshold different from the first threshold and the second threshold. The third threshold is preset by a user, for example. With this configuration, the setting unit 402 may set a guideline for reducing the calculation amount. For example, the setting unit 402 may enable detection that a solution of the parameter is unconverged.

Specifically, the setting unit 402 sets at least any one of the end condition A and the end condition C. Specifically, the setting unit 402 may set all of the end condition A, the end condition B, and the end condition C. Specifically, the setting unit 402 may set any two of the end condition A, the end condition B, and the end condition C.

The operation unit 403 repeatedly performs the specific operation. For example, the operation unit 403 repeatedly performs the specific operation until the end condition A is satisfied. Specifically, the operation unit 403 calculates a solution of the parameter using the function based on an initial value of the parameter. Thereafter, specifically, the operation unit 403 repeatedly performs the specific operation of calculating a solution of the parameter using the function based on the solution of the parameter calculated last time until the end condition A is satisfied. With this configuration, the operation unit 403 may accurately calculate a solution of the parameter. The operation unit 403 may suppress an increase in the calculation amount.

For example, the operation unit 403 repeatedly performs the specific operation until the end condition C is satisfied. Specifically, the operation unit 403 calculates a solution of the parameter using the function based on the initial value of the parameter. Thereafter, specifically, the operation unit 403 repeatedly performs the specific operation of calculating a solution of the parameter using the function based on the solution of the parameter calculated last time until the end condition C is satisfied. With this configuration, the operation unit 403 may facilitate detection that the solution of the parameter is unconverged relatively early. The operation unit 403 may suppress an increase in the calculation amount.

For example, the operation unit 403 may repeatedly perform the specific operation until at least any one of the end condition A and the end condition B is satisfied. Specifically, the operation unit 403 calculates a solution of the parameter using the function based on the initial value of the parameter. Thereafter, specifically, the operation unit 403 repeatedly performs the specific operation of calculating a solution of the parameter using the function based on the solution of the parameter calculated last time until at least any one of the end condition A and the end condition B is satisfied. With this configuration, the operation unit 403 may accurately calculate a solution of the parameter. The operation unit 403 may suppress an increase in the calculation amount.

For example, the operation unit 403 may repeatedly perform the specific operation until at least any one of the end condition C and the end condition B is satisfied. Specifically, the operation unit 403 calculates a solution of the parameter using the function based on the initial value of the parameter. Thereafter, specifically, the operation unit 403 repeatedly performs the specific operation of calculating a solution of the parameter using the function based on the solution of the parameter calculated last time until at least any one of the end condition C and the end condition B is satisfied. With this configuration, the operation unit 403 may accurately calculate a solution of the parameter. The operation unit 403 may facilitate detection that the solution of the parameter is unconverged relatively early. The operation unit 403 may suppress an increase in the calculation amount.

For example, the operation unit 403 may repeatedly perform the specific operation until at least any one of the end condition A and the end condition C is satisfied. Specifically, the operation unit 403 calculates a solution of the parameter using the function based on the initial value of the parameter. Thereafter, specifically, the operation unit 403 repeatedly performs the specific operation of calculating a solution of the parameter using the function based on the solution of the parameter calculated last time until at least any one of the end condition A and the end condition C is satisfied. With this configuration, the operation unit 403 may accurately calculate a solution of the parameter. The operation unit 403 may facilitate detection that the solution of the parameter is unconverged relatively early. The operation unit 403 may suppress an increase in the calculation amount.

For example, the operation unit 403 may repeatedly perform the specific operation until at least any one of the end condition A, the end condition B, and the end condition C is satisfied. Specifically, the operation unit 403 calculates a solution of the parameter using the function based on the initial value of the parameter. Thereafter, specifically, the operation unit 403 repeatedly performs the specific operation of calculating a solution of the parameter using the function based on the solution of the parameter calculated last time until at least any one of the end condition A, the end condition B, and the end condition C is satisfied. With this configuration, the operation unit 403 may accurately calculate a solution of the parameter. The operation unit 403 may facilitate detection that the solution of the parameter is unconverged relatively early. The operation unit 403 may suppress an increase in the calculation amount.

The determination unit 404 determines whether or not a solution of the parameter has converged. In a case where the end condition A is satisfied, the determination unit 404 determines that the solution of the parameter has converged. In a case where the end condition B is satisfied, the determination unit 404 determines that the solution of the parameter has converged. In a case where the end condition C is satisfied, the determination unit 404 determines that the solution of the parameter is unconverged.

The output unit 405 outputs a processing result of at least any one of the functional units. An output format is, for example, display on a display, print output to a printer, transmission to an external device by the network I/F 303, or storage to a storage area such as the memory 302 or the recording medium 305. With this configuration, the output unit 405 may make it possible to notify a user of the processing result of at least any one of the functional units, and may improve convenience of the information processing device 100.

For example, in a case where the end condition A is satisfied, the output unit 405 outputs the solution of the parameter calculated this time. Specifically, the output unit 405 outputs the solution of the parameter so that a user may refer to the solution. With this configuration, the output unit 405 may make a relatively accurate solution of the parameter available.

For example, in a case where the end condition B is satisfied, the output unit 405 outputs the solution of the parameter calculated this time. Specifically, the output unit 405 outputs the solution of the parameter so that a user may refer to the solution. With this configuration, the output unit 405 may make a relatively accurate solution of the parameter available.

For example, in a case where the end condition C is satisfied, the output unit 405 outputs a notification indicating that the solution of the parameter is unconverged. Specifically, the output unit 405 outputs the notification indicating that the solution of the parameter is unconverged so that a user may refer to the notification. With this configuration, the output unit 405 may enable the user to grasp that the solution of the parameter is unconverged.

### (First Operation Example of Information Processing Device 100)

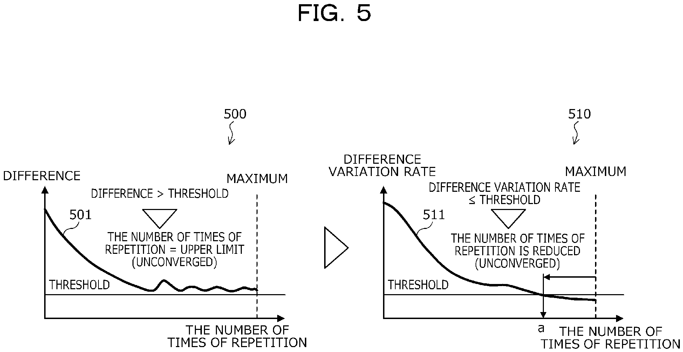

Next, a first operation example of the information processing device 100 will be described with reference to FIG. 5. The first operation example corresponds to a case where the information processing device 100 uses at least the end condition C described above.

FIG. 5 is an explanatory diagram illustrating the first operation example of the information processing device 100. In FIG. 5, the information processing device 100 repeatedly performs a specific operation of calculating an electron density p(r) of each point in a space in a predetermined situation setting according to the SCF method.

In the following description, the electron density p(r) calculated at the x-th time may be referred to as "electron density ρ^{x}(r)*"*. Furthermore, in the following description, it may be assumed that the electron density p(r) indicates the electron density p(r) at any one point in the space in order to simplify the description.

The information processing device 100 sets, for example, an end condition. The end condition includes, for example, that when an electron density ρⁿ(r) is calculated, a difference variation rate between a difference Δρⁿ⁻¹ (r) between an electron density ρⁿ⁻¹(r) and an electron density ρⁿ⁻²(r) and a difference Δρⁿ(r) between the electron density ρⁿ(r) and the electron density ρⁿ⁻¹(r) is equal to or smaller than a threshold DTH. DTH is, for example, 5 [%].

For example, the difference Δρⁿ⁻¹ (r) = electron density ρⁿ⁻²(r) - electron density ρⁿ⁻¹(r) is satisfied. For example, the difference Δρⁿ(r) = electron density ρⁿ⁻¹ (r) - electron density ρⁿ (r) is satisfied. The difference variation rate is (difference Δρⁿ(r) - difference Δρⁿ⁻¹ (r) ) /difference Δρⁿ⁻¹ (r). Specifically, in a case where there is a plurality of points in the space, the end condition includes that the difference variation rates of all the points are equal to or smaller than the threshold DTH.

The end condition may further include that the difference Δpⁿ(r) between the electron density ρⁿ(r) and the electron density ρⁿ⁻¹(r) is equal to or smaller than a Δρ allowable value. Specifically, in a case where there is a plurality of points in the space, the end condition may include that the difference Δρⁿ(r) of all the points are equal to or smaller than the Δρ allowable value.

When calculating the n-th electron density ρⁿ(r) and the difference variation rate is equal to or smaller than the threshold DTH, the information processing device 100 ends repetition of the specific operation and determines that the electron density ρⁿ(r) is unconverged. In a case where it is determined that the electron density ρⁿ(r) is unconverged, the information processing device 100 outputs a notification indicating that the electron density ρⁿ(r) is unconverged so that a user may refer to the notification.

When calculating the n-th electron density ρⁿ(r) and the difference Δpⁿ(r) is equal to or smaller than the Δρ allowable value, the information processing device 100 ends repetition of the specific operation and determines that the electron density ρⁿ(r) has converged. In a case where it is determined that the electron density ρⁿ(r) has converged, the information processing device 100 outputs the electron density ρⁿ(r) so that a user may refer to the electron density ρⁿ(r).

Furthermore, in a case where the number of times n reaches an upper limit, the information processing device 100 may end repetition of the specific operation and determine that the electron density ρⁿ(r) is unconverged.

On the other hand, a prior method adopts, as the end condition, that the difference Δρⁿ(r) between the electron density ρⁿ(r) and the electron density ρⁿ⁻¹(r) is equal to or smaller than the Δρ allowable value. Therefore, in the prior method, unless the difference Δpⁿ(r) becomes equal to or smaller than the Δρ allowable value, the specific operation is continuously repeatedly performed until the number of times n reaches the upper limit.

A graph 500 illustrates a characteristic curve 501 representing a correspondence relationship between the number of times n of repeating the specific operation and the difference Δρⁿ(r) for the prior method. As illustrated in the graph 500, in the prior method, in a situation setting in which the electron density ρⁿ(r) is difficult to converge, the specific operation is continuously repeatedly performed until the number of times n reaches the upper limit, which causes an increase in the calculation amount. In the prior method, when the upper limit of the number of times n is not set, an infinite loop is caused.

On the other hand, a graph 510 illustrates a characteristic curve 511 representing a correspondence relationship between the number of times n of repeating the specific operation and the difference variation rate for the information processing device 100. As illustrated in the graph 510, even in a situation setting in which the electron density ρⁿ(r) is difficult to converge, the information processing device 100 may relatively early determine that the electron density ρⁿ(r) is unconverged at the number of times n = a, and may end repetition of the specific operation. Therefore, the information processing device 100 may suppress an increase in the calculation amount.

Here, a case has been described where the information processing device 100 is applied to a problem of calculating the electron density p(r). However, the embodiment is not limited to this. For example, there may be a case where the information processing device 100 is applied to another problem other than the problem of calculating the electron density p(r) . Here, a case has been described where the information processing device 100 implements the SCF method. However, the embodiment is not limited to this. For example, there may be a case where the information processing device 100 implements an iterative solution other than the SCF method.

### (Overall Processing Procedure in First Operation Example)

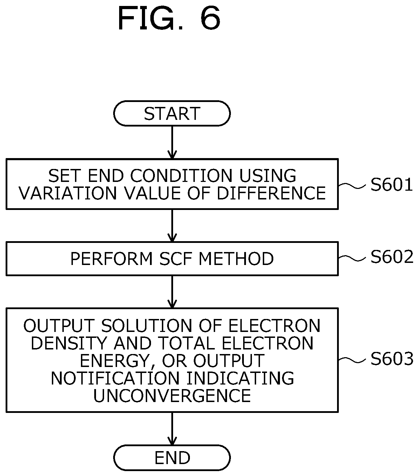

Next, an example of an overall processing procedure in the first operation example executed by the information processing device 100 will be described with reference to FIG. 6. The overall processing is implemented by, for example, the CPU 301, a storage area such as the memory 302 or the recording medium 305, and the network I/F 303 illustrated in FIG. 3.

FIG. 6 is a flowchart illustrating an example of the overall processing procedure in the first operation example. In FIG. 6, the information processing device 100 sets an end condition using a variation value of a difference (step S601). Next, the information processing device 100 performs the SCF method (Step S602). Then, the information processing device 100 outputs a solution of an electron density and total electron energy when the solution of the electron density has converged, and outputs a notification indicating unconvergence when the solution of the electron density is unconverged (step S603).

In step S603, specifically, in a case where there is a plurality of points in a space, when all solutions of the electron density at the plurality of points have converged, the information processing device 100 outputs the solution of the electron density at each point and the total electron energy. Specifically, in the case where there is the plurality of points in the space, the information processing device 100 outputs the notification indicating unconvergence when the solution of the electron density of at least any one of the points is unconverged. Thereafter, the information processing device 100 ends the overall processing.

### (Operation Processing Procedure in First Operation Example)

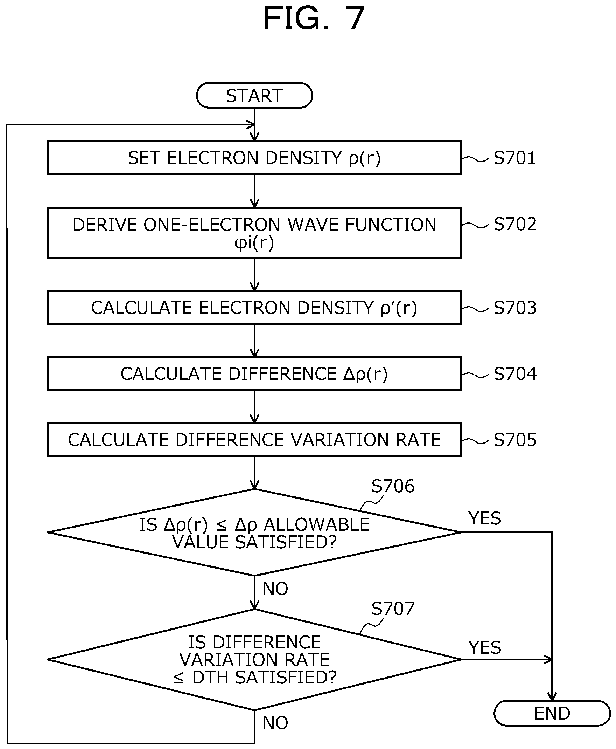

Next, an example of an operation processing procedure in the first operation example executed by the information processing device 100 will be described with reference to FIG. 7. Operation processing is implemented by, for example, the CPU 301, a storage area such as the memory 302 or the recording medium 305, and the network I/F 303 illustrated in FIG. 3.

FIG. 7 is a flowchart illustrating an example of the operation processing procedure in the first operation example. In FIG. 7, the information processing device 100 sets an electron density p(r) (step S701). For example, in a case where there is a plurality of points in a space, the information processing device 100 sets the electron density p(r) of each point. For example, the information processing device 100 sets an initial value of the electron density p(r) in the first setting, and sets the electron density p(r) by some method in the second and subsequent settings.

Next, the information processing device 100 solves a Kohn-Sham equation based on the electron density p(r), and derives a one-electron wave function ϕi (r) (step S702). For example, in the case where there is the plurality of points in the space, the information processing device 100 derives the one-electron wave function ϕi (r) for each point. Then, the information processing device 100 calculates an electron density p' (r) using the one-electron wave function ϕi (r) (step S703). For example, in the case where there is the plurality of points in the space, the information processing device 100 calculates the electron density p'(r) of each point.

Next, the information processing device 100 calculates a difference Δρ (r) = electron density p(r) - electron density p' (r) (step S704). Then, the information processing device 100 calculates a difference variation rate = (current difference Δρ (r) - previous difference Δp(r))/previous difference Δρ (r) (step S705). For example, in the case where there is the plurality of points in the space, the information processing device 100 calculates the difference Δρ (r) and the difference variation rate of each point.

Next, the information processing device 100 determines whether or not the difference Δρ (r) ≤ Δρ allowable value is satisfied (step S706). For example, in the case where there is the plurality of points in the space, the information processing device 100 determines whether or not the difference Δρ (r) of all the points ≤ Δρ allowable value is satisfied.

Here, in a case where the difference Δρ (r) ≤ Δρ allowable value is satisfied (step S706: Yes), the information processing device 100 determines that the electron density p(r) has converged, outputs the electron density p(r), and ends the operation processing. For example, in the case where there is the plurality of points in the space, when the difference Δρ (r) of all the points ≤ Δρ allowable value is satisfied, the information processing device 100 ends the operation processing.

On the other hand, in a case where the difference Δρ (r) ≤ Δρ allowable value is not satisfied (step S706: No), the information processing device 100 proceeds to processing of step S707. For example, in the case where there is the plurality of points in the space, when the difference Δρ (r) of at least any one of the points ≤ Δρ allowable value is not satisfied, the information processing device 100 proceeds to the processing of step S707.

In step S707, the information processing device 100 determines whether or not the difference variation rate ≤ DTH is satisfied (step S707). For example, in the case where there is the plurality of points in the space, the information processing device 100 determines whether or not the difference variation rate of at least any one of the points ≤ DTH is satisfied.

Here, in a case where the difference variation rate ≤ DTH is satisfied (step S707: Yes), the information processing device 100 determines that the electron density p(r) is unconverged, outputs a notification of unconvergence, and ends the operation processing. For example, in the case where there is the plurality of points in the space, when the difference variation rate of at least any one of the points ≤ DTH is satisfied, the information processing device 100 ends the operation processing.

On the other hand, in a case where the difference variation rate ≤ DTH is not satisfied (step S707: No), the information processing device 100 returns to the processing of step S701. For example, in the case where there is the plurality of points in the space, when the difference variation rate of all the points ≤ DTH is not satisfied, the information processing device 100 returns the processing of step S701.

Here, the information processing device 100 may swap some steps in the processing order in each of the flowcharts of FIGs. 6 and 7 and execute the processing. For example, steps S706 and S707 may be swapped in the processing order. Furthermore, the information processing device 100 may omit the processing in some steps in each of the flowcharts of FIGs. 6 and 7. For example, the processing of step S706 may be omitted.

### (Second Operation Example of Information Processing Device 100)

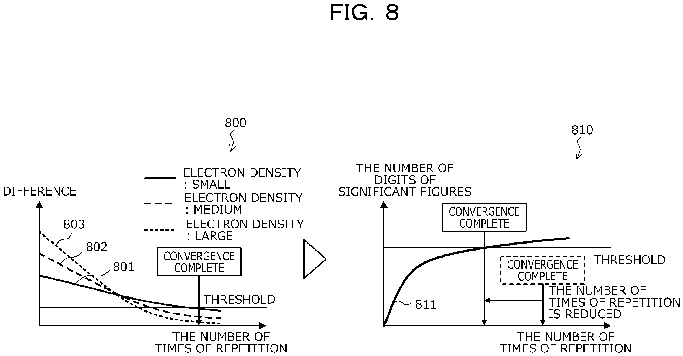

Next, a second operation example of the information processing device 100 will be described with reference to FIG. 8. The second operation example corresponds to a case where the information processing device 100 uses at least the end condition A described above.

FIG. 8 is an explanatory diagram illustrating the second operation example of the information processing device 100. In FIG. 8, the information processing device 100 repeatedly performs a specific operation of calculating an electron density p(r) of each point in a space in a predetermined situation setting according to the SCF method.

In the following description, the electron density p(r) calculated at the x-th time may be referred to as "electron density ρ^{x}(r)*"*. Furthermore, in the following description, it may be assumed that the electron density p(r) indicates the electron density p(r) at any one point in the space in order to simplify the description.

The information processing device 100 sets, for example, an end condition. The end condition includes, for example, that when an electron density ρⁿ(r) is calculated, the number of digits of significant figures is equal to or greater than a threshold SD between total electron energy corresponding to an electron density ρⁿ⁻¹(r) and total electron energy corresponding to the electron density ρⁿ(r). SD is, for example, 5 [digits].

The significant figure is a number string of a portion in which values match in a case where the values in the same digits successively match from upper digits. The number of digits of significant figures is the number indicating how many digits have matching values in the case where the values in the same digits successively match from the upper digits.

The end condition may include, for example, that when the electron density ρⁿ(r) is calculated, the number of digits of significant figures of the past i or more consecutive times up to this time is equal to or greater than the threshold SD. For example, i is an integer equal to or greater than 2.

The end condition may further include, for example, that a difference Δpⁿ(r) between the electron density ρⁿ(r) and the electron density ρⁿ⁻¹(r) is equal to or smaller than a Δρ allowable value. Specifically, in a case where there is a plurality of points in the space, the end condition may include that the difference Δρⁿ(r) of all the points are equal to or smaller than the Δρ allowable value.

When calculating the n-th electron density ρⁿ(r) and the number of digits of significant figures is equal to or greater than SD, the information processing device 100 ends repetition of the specific operation and determines that the electron density ρⁿ(r) has converged. In a case where it is determined that the electron density ρⁿ(r) has converged, the information processing device 100 outputs the electron density ρⁿ(r) so that a user may refer to the electron density ρⁿ (r) .

When calculating the n-th electron density ρⁿ(r) and the difference Δpⁿ(r) is equal to or smaller than the Δρ allowable value, the information processing device 100 ends repetition of the specific operation and determines that the electron density ρⁿ(r) has converged. In a case where it is determined that the electron density ρⁿ(r) has converged, the information processing device 100 outputs the electron density ρⁿ(r) so that a user may refer to the electron density ρⁿ(r).

Furthermore, in a case where the number of times n reaches an upper limit, the information processing device 100 may end repetition of the specific operation and determine that the electron density ρⁿ(r) is unconverged.

On the other hand, a prior method adopts, as the end condition, that the difference Δρⁿ (r) between the electron density ρⁿ(r) and the electron density ρⁿ⁻¹(r) is equal to or smaller than the Δρ allowable value. Therefore, in the prior method, unless the difference Δpⁿ(r) becomes equal to or smaller than the Δρ allowable value, the specific operation is continuously repeatedly performed until the number of times n reaches the upper limit.

A graph 800 illustrates characteristic curves 801 to 803 representing a correspondence relationship between the number of times n of repeating the specific operation and a difference Δpⁿ(rᵢ) for a point i in the space, for the prior method. Specifically, the characteristic curve 801 corresponds to the point i at which an electron density pⁿ(rᵢ) is relatively small. Specifically, the characteristic curve 802 corresponds to the point i at which the electron density pⁿ(rᵢ) is medium. Specifically, the characteristic curve 803 corresponds to the point i at which the electron density pⁿ(rᵢ) is relatively large.

As illustrated in the graph 800, in the prior method, in a situation setting in which the electron density pⁿ(rᵢ) at the any one point i is relatively small and a degree of decrease in the difference Δpⁿ (rᵢ) is relatively small, the number of times n of repeating the specific operation easily increases, causing an increase in the calculation amount.

On the other hand, a graph 810 illustrates a characteristic curve 811 representing a correspondence relationship between the number of times n of repeating the specific operation and the number of digits of significant figures for the information processing device 100. As illustrated in the graph 810, the information processing device 100 may relatively early determine that the electron density pⁿ(rᵢ) has converged even in a situation setting in which the electron density pⁿ(rᵢ) at any one of the points is relatively small and the degree of decrease in the difference Δρⁿ(rᵢ) is relatively small. Then, the information processing device 100 may end repetition of the specific operation. Therefore, the information processing device 100 may suppress an increase in the calculation amount.

Here, a case has been described where the information processing device 100 is applied to a problem of calculating the electron density p(r). However, the embodiment is not limited to this. For example, there may be a case where the information processing device 100 is applied to another problem other than the problem of calculating the electron density ρ(r). Here, a case has been described where the information processing device 100 implements the SCF method. However, the embodiment is not limited to this. For example, there may be a case where the information processing device 100 implements an iterative solution other than the SCF method.

### (Overall Processing Procedure in Second Operation Example)

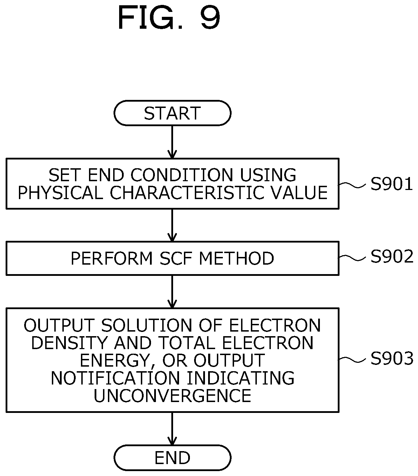

Next, an example of an overall processing procedure in the second operation example executed by the information processing device 100 will be described with reference to FIG. 9. The overall processing is implemented by, for example, the CPU 301, a storage area such as the memory 302 or the recording medium 305, and the network I/F 303 illustrated in FIG. 3.

FIG. 9 is a flowchart illustrating an example of the overall processing procedure in the second operation example. In FIG. 9, the information processing device 100 sets an end condition using a physical characteristic value (step S901). Next, the information processing device 100 performs the SCF method (Step S902). Then, the information processing device 100 outputs a solution of an electron density and total electron energy when the solution of the electron density has converged, and outputs a notification indicating unconvergence when the solution of the electron density is unconverged (step S903).

In step S903, specifically, in a case where there is a plurality of points in a space, when all solutions of the electron density at the plurality of points have converged, the information processing device 100 outputs the solution of the electron density at each point and the total electron energy. Specifically, in the case where there is the plurality of points in the space, the information processing device 100 outputs the notification indicating unconvergence when the solution of the electron density of at least any one of the points is unconverged. Thereafter, the information processing device 100 ends the overall processing.

### (Operation Processing Procedure in Second Operation Example)

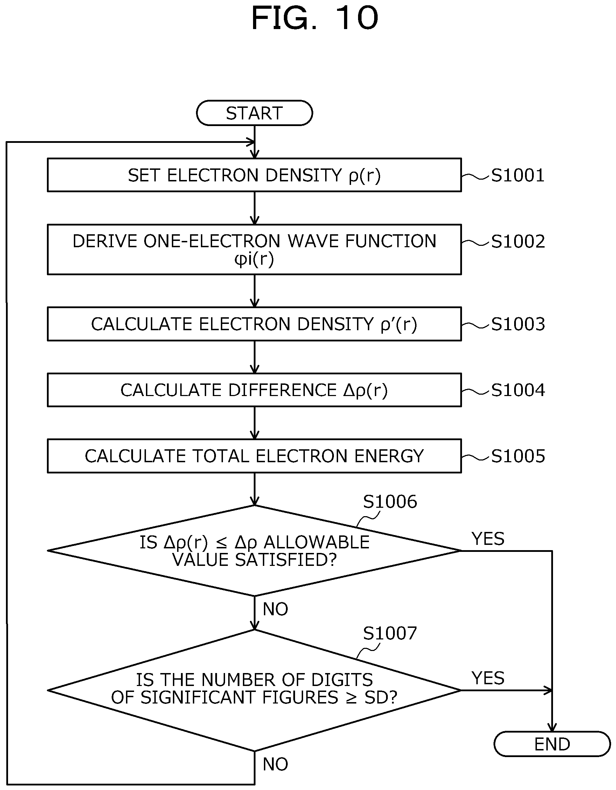

Next, an example of an operation processing procedure in the second operation example executed by the information processing device 100 will be described with reference to FIG. 10. Operation processing is implemented by, for example, the CPU 301, a storage area such as the memory 302 or the recording medium 305, and the network I/F 303 illustrated in FIG. 3.

FIG. 10 is a flowchart illustrating an example of the operation processing procedure in the second operation example. In FIG. 10, the information processing device 100 sets an electron density p(r) (step S1001). For example, in a case where there is a plurality of points in a space, the information processing device 100 sets the electron density p(r) of each point. For example, the information processing device 100 sets an initial value of the electron density p(r) in the first setting, and sets the electron density p(r) by some method in the second and subsequent settings.

Next, the information processing device 100 solves a Kohn-Sham equation based on the electron density p(r), and derives a one-electron wave function ϕi(r) (step S1002). For example, in the case where there is the plurality of points in the space, the information processing device 100 derives the one-electron wave function ϕi(r) for each point. Then, the information processing device 100 calculates an electron density p' (r) using the one-electron wave function ϕi(r) (step S1003). For example, in the case where there is the plurality of points in the space, the information processing device 100 calculates the electron density p' (r) of each point.

Next, the information processing device 100 calculates a difference Δρ(r) = electron density p(r) - electron density p' (r) (step S1004). For example, in the case where there is the plurality of points in the space, the information processing device 100 calculates the difference Δρ(r) of each point. Then, the information processing device 100 calculates total electron energy as a physical characteristic value based on the electron density p' (r) (step S1005) .

Next, the information processing device 100 determines whether or not the difference Δρ(r) ≤ Δρ allowable value is satisfied (step S1006). For example, in the case where there is the plurality of points in the space, the information processing device 100 determines whether or not the difference Δρ(r) of all the points ≤ Δρ allowable value is satisfied.

Here, in a case where the difference Δρ(r) ≤ Δρ allowable value is satisfied (step S1006: Yes), the information processing device 100 determines that the electron density p(r) has converged, outputs the electron density p(r), and ends the operation processing. For example, in the case where there is the plurality of points in the space, when the difference Δρ(r) of all the points ≤ Δρ allowable value is satisfied, the information processing device 100 ends the operation processing.

On the other hand, in a case where the difference Δρ(r) ≤ Δρ allowable value is not satisfied (step S1006: No), the information processing device 100 proceeds to processing of step S1007. For example, in the case where there is the plurality of points in the space, when the difference Δρ(r) of at least any one of the points ≤ Δρ allowable value is not satisfied, the information processing device 100 proceeds to the processing of step S1007.

In step S1007, the information processing device 100 determines whether or not the number of digits of significant figures of the total electron energy ≥ SD is satisfied (step S1007). Here, in a case where the number of digits of significant figures ≥ SD is satisfied (step S1007: Yes), the information processing device 100 determines that the electron density p(r) has converged, outputs the electron density p(r), and ends the operation processing. On the other hand, in a case where the number of digits of significant figures ≥ SD is not satisfied (step S1007: No), the information processing device 100 returns to the processing of step S1001.

Here, the information processing device 100 may swap some steps in the processing order in each of the flowcharts of FIGs. 9 and 10 and execute the processing. For example, steps S1006 and S1007 may be swapped in the processing order. Furthermore, the information processing device 100 may omit the processing in some steps in each of the flowcharts of FIGs. 9 and 10. For example, the processing of step S1006 may be omitted.

### (Effect of Information Processing Device 100)

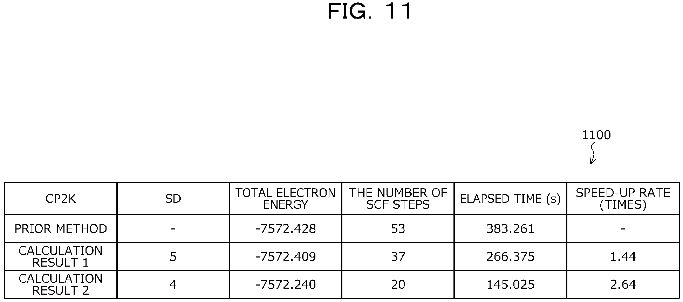

Next, an example of an effect of the information processing device 100 will be described with reference to FIGs. 11 to 13.

FIG. 11 is an explanatory diagram illustrating a comparative example of a calculation result with a prior method. A table 1100 in FIG. 11 illustrates, for a problem of an ammonia catalyst, a calculation result of the prior method using CP2K, a calculation result 1 in a case where SD = 5 is set using the CP2K in the second operation example, and a calculation result 2 in a case where SD = 4 is set using the CP2K in the second operation example. The CP2K is open source software.

In the prior method, total electron energy = -7572.428 is calculated. In the prior method, the number of steps when the total electron energy = -7572.428 is calculated is 53 steps. In the prior method, an elapsed time at the time of calculating the total electron energy = -7572.428 = calculation time is 383.261 s.

On the other hand, in the calculation result 1, total electron energy = -7572.409. In the calculation result 1, the number of steps when the total electron energy = -7572.409 is calculated is 37 steps. In the calculation result 1, an elapsed time at the time of calculating the total electron energy = -7572.409 = calculation time is 266.375 s. In the calculation result 1, a speed-up rate of the calculation time based on the prior method is 1.44. As indicated in the calculation result 1, the information processing device 100 may calculate a value of the total electron energy similar to that of the prior method at 1.44 times higher speed than that of the prior method.

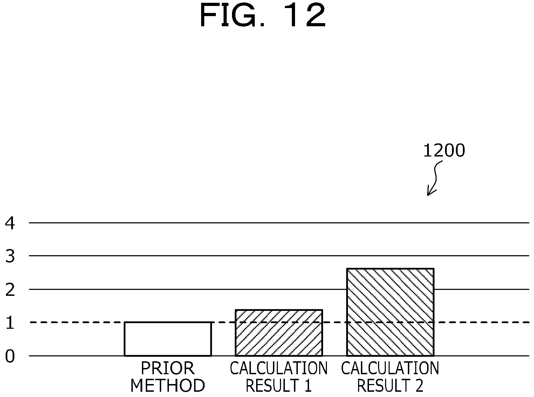

Furthermore, in the calculation result 2, total electron energy = -7572.240. In the calculation result 2, the number of steps when the total electron energy = -7572.240 is calculated is 20 steps. In the calculation result 2, an elapsed time at the time of calculating the total electron energy = -7572.240 = calculation time is 145.025 s. In the calculation result 2, a speed-up rate of the calculation time based on the prior method is 2.64. As indicated in the calculation result 2, the information processing device 100 may calculate a value of the total electron energy similar to that of the prior method at 2.64 times higher speed than that of the prior method. Next, description of FIG. 12 will be made.

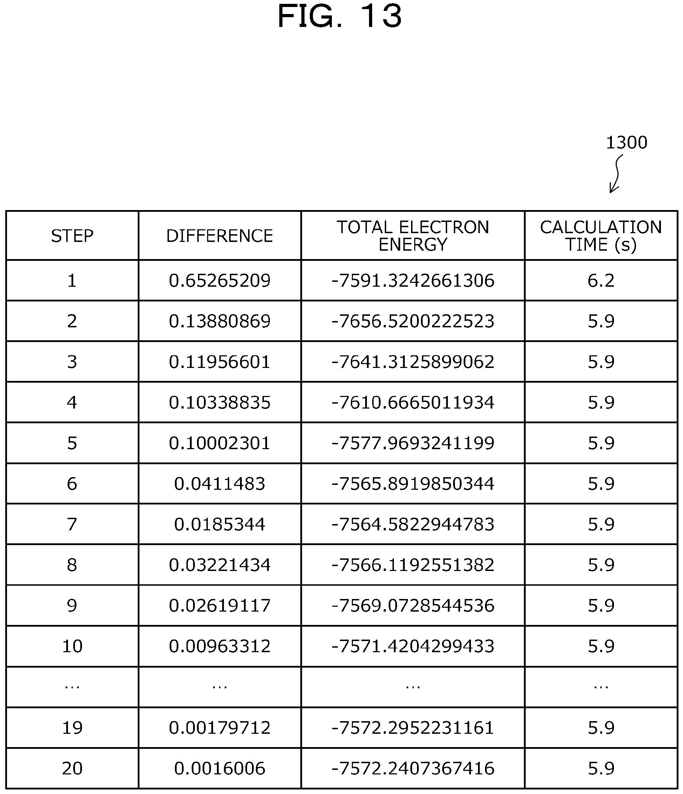

FIG. 12 is an explanatory diagram illustrating the comparative example of the speed-up rate with the prior method. A graph 1200 of FIG. 12 illustrates the speed-up rate of the calculation result 1 and the speed-up rate of the calculation result 2 in a case where the prior method is used as a reference: 1. As illustrated in the graph 1200, the information processing device 100 may speed up the SCF method as compared with the prior method, and may suppress an increase in the calculation time needed for the SCF method. The information processing device 100 may achieve, for example, 1.4 to 2.6 times higher speed than the prior method. Next, description of FIG. 13 will be made.

FIG. 13 is an explanatory diagram illustrating a display example. In FIG. 13, the information processing device 100 outputs a log 1300 indicating a calculation result for each step. In the example of FIG. 13, specifically, the information processing device 100 outputs the log 1300 indicating a calculation result for each step corresponding to the calculation result 2. The log 1300 indicates, for each step, the number of steps in the step, a difference in the step, total electron energy in the step, and a calculation time in the step in association with each other.

In this manner, the information processing device 100 may determine that the electron density p(r) and the total electron energy have converged before the difference Δρ(r) ≤ Δρ allowable value is satisfied based on the number of significant digits, and may suppress an increase in the calculation amount.

As described above, according to the information processing device 100, it is possible to repeatedly perform a specific operation until the number of upper digits whose values match between a characteristic value corresponding to a solution of a parameter calculated last time and a characteristic value corresponding to a solution of the parameter calculated this time becomes equal to or greater than a first threshold. With this configuration, the information processing device 100 may suppress an increase in the calculation amount.

According to the information processing device 100, in a case where the number of digits becomes equal to or greater than the first threshold, it is possible to output the solution of the parameter calculated this time. With this configuration, the information processing device 100 may make the solution of the parameter available.

According to the information processing device 100, it is possible to repeatedly perform the specific operation until the number of digits becomes equal to or greater than the first threshold or a difference between the solution of the parameter calculated last time and the solution of the parameter calculated this time becomes equal to or smaller than a second threshold. With this configuration, the information processing device 100 may suppress an increase in the calculation amount. The information processing device 100 may detect that a relatively accurate solution of the parameter has been calculated.

According to the information processing device 100, it is possible to calculate the solution of the parameter using a function based on an initial value of the parameter. According to the information processing device 100, it is possible to repeatedly perform a specific operation of calculating the solution of the parameter using the function based on the solution of the parameter calculated last time until the number of digits becomes equal to or greater than the first threshold. With this configuration, the information processing device 100 may make it possible to calculate the solution of the parameter.

According to the information processing device 100, it is possible to repeatedly perform a specific operation until a degree of variation between a difference between a solution of the parameter calculated last time and a solution of the parameter calculated a time before last, and a difference between a solution of the parameter calculated this time and the solution of the parameter calculated last time becomes equal to or smaller than a third threshold. With this configuration, the information processing device 100 may suppress an increase in the calculation amount.

According to the information processing device 100, in a case where the degree of variation becomes equal to or smaller than the third threshold, it is possible to output a notification indicating that the solution of the parameter is unconverged. With this configuration, the information processing device 100 may enable a user to grasp that the solution of the parameter is unconverged.

According to the information processing device 100, it is possible to repeatedly perform the specific operation until the degree of variation becomes equal to or smaller than the third threshold or the difference between the solution of the parameter calculated last time and the solution of the parameter calculated this time becomes equal to or smaller than the second threshold. With this configuration, the information processing device 100 may suppress an increase in the calculation amount. The information processing device 100 may detect that a relatively accurate solution of the parameter has been calculated.

According to the information processing device 100, it is possible to calculate the solution of the parameter using a function based on an initial value of the parameter. According to the information processing device 100, it is possible to repeatedly perform the specific operation of calculating the solution of the parameter using the function based on the solution of the parameter calculated last time until the degree of variation becomes equal to or smaller than the third threshold. With this configuration, the information processing device 100 may make it possible to calculate the solution of the parameter.

According to the information processing device 100, it is possible to repeatedly perform the specific operation until the number of digits becomes equal to or greater than the first threshold or the degree of variation becomes equal to or smaller than the third threshold. With this configuration, the information processing device 100 may suppress an increase in the calculation amount.

According to the information processing device 100, it is possible to adopt an electron density as the parameter. According to the information processing device 100, it is possible to include, in the function, a Kohn-Sham equation that enables calculation of a one-electron wave function. With this configuration, the information processing device 100 may suppress an increase in the calculation amount when the electron density is calculated.

According to the information processing device 100, it is possible to adopt an operation corresponding to the SCF method as the specific operation. With this configuration, the information processing device 100 may speed up the SCF method.

Note that the information processing method described in the present embodiment may be implemented by executing a program prepared in advance in a computer such as a PC or a workstation. The information processing program described in the present embodiment is executed by being recorded in a computer-readable recording medium and being read from the recording medium by the computer. The recording medium is a hard disk, a flexible disk, a compact disc (CD)-ROM, a magneto optical disc (MO), a digital versatile disc (DVD), or the like. Furthermore, the information processing program described in the present embodiment may be distributed via a network such as the Internet.

### REFERENCE SIGNS LIST

100 Information processing device
101 Function
200 Information processing system
201 Numerical calculation device
202 Client device
210 Network
300 Bus
301 CPU
302 Memory
303 Network I/F
304 Recording medium I/F
305 Recording medium
400 Storage unit
401 Acquisition unit
402 Setting unit
403 Operation unit
404 Determination unit
405 Output unit
500, 510, 800, 810, 1200 Graph
501, 511, 801 to 803, 811 Characteristic curve
1100 Table
1300 Log

## Claims

1. An information processing program for causing a computer to execute processing comprising
repeatedly performing a specific operation of calculating a solution of a parameter by using a function until the number of upper digits whose values match between a characteristic value that corresponds to a solution of the parameter calculated last time and a characteristic value that corresponds to a solution of the parameter calculated this time becomes equal to or greater than a first threshold.

2. The information processing program according to claim 1, for causing the computer to further execute processing comprising
outputting, in a case where the number of digits becomes equal to or greater than the first threshold, the solution of the parameter calculated this time.

3. The information processing program according to claim 1 or 2, wherein,
in the processing of performing,
the specific operation is repeatedly performed until the number of digits becomes equal to or greater than the first threshold or a difference between the solution of the parameter calculated last time and the solution of the parameter calculated this time becomes equal to or smaller than a second threshold.

4. The information processing program according to claim 1 or 2, wherein,
after causing the computer to execute processing of
calculating the solution of the parameter by using the function based on an initial value of the parameter,
in the processing of performing,
a specific operation of calculating the solution of the parameter by using the function based on the solution of the parameter calculated last time is repeatedly performed until the number of digits becomes equal to or greater than the first threshold.

5. An information processing program for causing a computer to execute processing comprising
repeatedly performing a specific operation of calculating a solution of a parameter by using a function until a degree of variation between a difference between a solution of the parameter calculated last time and a solution of the parameter calculated a time before last, and a difference between a solution of the parameter calculated this time and the solution of the parameter calculated last time becomes equal to or smaller than a first threshold.

6. The information processing program according to claim 5, for causing the computer to further execute processing comprising
outputting, in a case where the degree of variation becomes equal to or smaller than the first threshold, a notification that indicates that the solution of the parameter is unconverged.

7. The information processing program according to claim 5 or 6, wherein,
in the processing of performing,
the specific operation is repeatedly performed until the degree of variation becomes equal to or smaller than the first threshold or the difference between the solution of the parameter calculated last time and the solution of the parameter calculated this time becomes equal to or smaller than a second threshold.

8. The information processing program according to claim 5 or 6, wherein,
after causing the computer to execute processing of
calculating the solution of the parameter by using the function based on an initial value of the parameter,
in the processing of performing,
a specific operation of calculating the solution of the parameter by using the function based on the solution of the parameter calculated last time is repeatedly performed until the degree of variation becomes equal to or smaller than the first threshold.

9. The information processing program according to claim 1 or 2, wherein,
in the processing of performing,
the specific operation is repeatedly performed until the number of digits becomes equal to or greater than the first threshold or a degree of variation between a difference between the solution of the parameter calculated last time and a solution of the parameter calculated a time before last, and a difference between the solution of the parameter calculated this time and the solution of the parameter calculated last time becomes equal to or smaller than a third threshold.

10. The information processing program according to any one of claims 1, 2, 5, and 6, wherein
the parameter is an electron density, and
the function includes a Kohn-Sham equation that enables calculation of a one-electron wave function.

11. The information processing program according to any one of claims 1, 2, 5, and 6, wherein the specific operation corresponds to a self consistent field method.

12. An information processing method for causing a computer to execute processing comprising
repeatedly performing a specific operation of calculating a solution of a parameter by using a function until the number of upper digits whose values match between a characteristic value that corresponds to a solution of the parameter calculated last time and a characteristic value that corresponds to a solution of the parameter calculated this time becomes equal to or greater than a first threshold.

13. An information processing method for causing a computer to execute processing comprising
repeatedly performing a specific operation of calculating a solution of a parameter by using a function until a degree of variation between a difference between a solution of the parameter calculated last time and a solution of the parameter calculated a time before last, and a difference between a solution of the parameter calculated this time and the solution of the parameter calculated last time becomes equal to or smaller than a first threshold.
